**Europäisches Patentamt**

**European Patent Office**

**Office européen des brevets**

(11) Numéro de publication: **0 264 305**
**A2**

# (12) DEMANDE DE BREVET EUROPEEN

(21) Numéro de dépôt: **87401910.2**

(22) Date de dépôt: **19.08.87**

(51) Int. Cl.⁴: **C 12 Q 1/68**
**// C12N15/00**

(30) Priorité: **19.08.86 FR 8611859**

(43) Date de publication de la demande:
**20.04.88 Bulletin 88/16**

(84) Etats contractants désignés:
**AT BE CH DE ES FR GB GR IT LI LU NL SE**

(71) Demandeur: **Vassart, Gilbert**
**113 Avenue Lambeau**
**B-1200 Bruxelles (BE)**

**Dumont, Jacques**
**32 Chemin du Chêne au Renard**
**B-1328 Ohain (BE)**

**Christophe, Daniel**
**71 rue des Héros**
**B-7199 Henripont (BE)**

**Georges, Michel**
**Deurnestraat 170**
**B-2510 Mortsel (BE)**

(72) Inventeur: **Vassart, Gilbert**
**113 Avenue Lambeau**
**B-1200 Bruxelles (BE)**

**Dumont, Jacques**
**32 Chemin du Chêne au Renard**
**B-1328 Ohain (BE)**

**Christophe, Daniel**
**71 rue des Héros**
**B-7199 Henripont (BE)**

**Georges, Michel**
**Deurnestraat 170**
**B-2510 Mortsel (BE)**

(74) Mandataire: **Gutmann, Ernest et al**
**S.C. Ernest Gutmann - Yves Plasseraud 67, boulevard**
**Haussmann**
**F-75008 Paris (FR)**

Le titre de l'invention a été modifié (Directives relatives à l'examen pratiqué à l'OEB, A-III, 7.3)

(54) **Sonde d'hybridation pour la détection de séquences nucléotidiques polymorphes.**

(57) L'invention concerne un nouveau fragment d'ADN utilisable en tant que sonde d'hybridation pour la détection de séquences d'ADN polymorphes humaines, animales et/ou végétales, et pour la détermination des degrés de parenté des individus dont elles provenaient.

La sonde d'hybridation contient des régions mini-satellites provenant du phage M13 et le procédé d'hybridation est mis en oeuvre en l'absence d'ADNs de compétition.

EP 0 264 305 A2

Bundesdruckerei Berlin

## Description

SONDE D'HYBRIDATION POUR LA DETECTION DE SEQUENCES NUCLEOTIDIQUES POLYMORPHES CONTENUES DANS UN ECHANTILLON D'ADN HUMAIN, ANIMAL OU VEGETAL, PROCEDE DE DETECTION DE SEQUENCES D'ADN POLYMORPHES UTILISANT UNE TELLE SONDE, SES APPLICATIONS BIOLOGIQUES.

L'invention concerne une nouvelle sonde d'ADN pour la détection de séquences nucléotidiques polymorphes à partir d'un aliquot d'ADN humain, animal ou végétal.

L'invention concerne également un nouveau procédé de détection de séquences d'ADN polymorphes utilisant une telle sonde, ainsi que ses applications biologiques.

Dans ce qui suit, on désignera par ADN toute séquence nucléotidique d'origine humaine, animale ou végétale.

La possibilité de déterminer avec certitude qu'un échantillon d'ADN est issu du génome d'un individu particulier revêt aujourd'hui une grande importance. En effet, dans de très nombreux cas, il est essentiel de connaître (avec exactitude) l'origine d'un échantillon d'ADN. Ainsi, dans l'élevage de races animales, il est important de connaître la paternité des animaux élevés afin de pouvoir déterminer leur pedigree.

Le détermination de l'origine d'un échantillon d'ADN humain est également importante. Il peut, en effet, s'avérer utile dans certains cas, de confirmer ou d'infirmer les liens de parenté supposés d'individus entre eux.

De même, l'identification d'un sujet à partir d'un aliquot d'ADN est essentielle dans certaines circonstances, en particulier pour la médecine légale.

Récemment, on a mis en évidence dans le génome humain des régions dites hypervariables (Réf. 1, 2). Ces régions hypervariables, désignées dans ce qui suit par le terme "mini-satellite", sont constituées de séquences répétées en "tandem", dispersées dans le génome.

On entend par l'expression : "séquences répétées en tandem", des séquences se jouxtant l'une à l'autre un certain nombre de fois dans le génome, cette répétition s'effectuant de façon monotone, la fin d'une séquence étant suivie du début de l'autre.

Chaque mini-satellite est donc constitué d'une séquence nucléotidique, généralement courte et désignée dans ce qui suit par le terme "motif unitaire", cette séquence nucléotidique étant répétée en "tandem" à différents endroits du génome.

Il a été montré dans la publication référencée en (2) ci-dessus, que ces mini-satellites sont polymorphes en ce que les nombres de motifs unitaires répétés contenus dans ces mini-satellites varient selon les individus. C'est donc ce polymorphisme, plutôt que la composition des séquences nucléotidiques contenues dans ces régions, qui est à la base de leur "hypervariabilité".

En effet, pour un lieu donné dans le génome humain et pour deux individus différents, le motif unitaire est répété un nombre différent de fois.

Les recherches poursuivies dans ce domaine ont montré d'autre part que les polymorphismes associés aux mini-satellites se transmettent de façon mendélienne et sont spécifiques d'un individu . Ceci étant, ils peuvent être utilisés pour déterminer l'origine d'un fragment d'ADN humain (Réf. 3, 4). On a ainsi proposé dans les articles référencés ci-dessus, de préparer des sondes contenant une séquence mini-satellite issue du génome humain, notamment du gène de la myoglobine.

L'hybridation de cette sonde, marquée radioactivement, avec un échantillon d'ADN humain fragmenté, séparé par électrophorèse et fixé sur un support, permet alors d'obtenir des images ou "patterns' de l'hybridation de la sonde avec les séquences d'ADN complémentaires présentes dans l'échantillon.

En comparant les positions, les nombres et les tailles des bandes obtenues à partir de fragments d'ADN provenant de deux échantillons distincts, on peut déterminer si ces échantillons sont issus du même génome, de génomes provenant de personnes directement parentes entre elles ou sans lien de parenté direct.

On peut, de la même façon, déterminer les éventuels liens de parenté, ou le pedigree, entre différents individus, par comparaison des positions, du nombre et de l'importance des bandes obtenues.

Dans ces expériences, la séquence d'ADN est d'abord isolée à partir du génome humain, et clonée dans un vecteur avant d'être utilisée dans un procédé d'hybridation.

Il était connu d'autre part depuis longtemps que le bactériophage M13 constitue un excellent vecteur, notamment pour des expériences de clonage de séquences nucléotidiques et de séquençage selon la méthode décrite par Sanger (Réf. 5).

L'invention se propose de remédier en grande partie aux inconvénients de l'art antérieur,notamment en ce qui concerne l'isolement et l'utilisation de séquences d'ADN particulières en vue de la détection de séquences d'ADN polymorphes.

Les recherches entreprises par les Inventeurs dans ce domaine leur ont en effet permis de trouver une nouvelle séquence nucléotidique utilisable en tant que sonde, isolable à partir d'un organisme non humain couramment utilisé dans les laboratoires, d'un emploi simple et donnant des résultats d'une grande qualité.

L'invention a pour premier objet une telle nouvelle sonde.

L'invention a pour second objet un nouveau procédé de détection de séquences d'ADN polymorphes utilisant une telle sonde.

L'invention a enfin pour objet les applications biologiques de la sonde selon l'invention.

La sonde nucléotidique selon l'invention, pour la détection de séquences d'ADN polymorphes humaines, animales et/ou végétales, se caractérise en ce qu'elle est constituée par un ADN issu du bactériophage M13 et en ce qu'elle comprend au moins un motif unitaire répété de façon monotone, chaque motif étant constitué par une séquence nucléotidique contenant la séquence suivante :

G-A-G-G-G-T-G-G-X-G-G-X-T-C-T
dans laquelle X représente la thymine, la cytosine, l'adénine ou la guanine, avantageusement la thymine ou la cytosine.

Selon une forme de réalisation préférée de l'invention, la sonde nucléotidique se caractérise en ce qu'elle est constituée par un ADN réplicable issu du bactériophage M13.

D'une manière avantageuse, le fragment d'ADN selon l'invention est isolé à partir du génome du bactériophage sauvage M13, notamment à partir de la séquence nucléotidique comprenant le gène codant pour la protéine III du bactériophage M13.

La sonde de l'invention contenant un ADN réplicable issu du bactériophage M13 constitue alors une sonde réplicable. En effet, l'ADN viral du phage M13, lorsqu'il est introduit dans une bactérie E.coli, peut s'y répliquer et donner naissance à de nouvelles particules virales.

Le cas échéant, les parties non essentielles à la réplication du phage M13 peuvent être délétées ou substituées par un autre fragment d'ADN.

La propriété que possèdent les virus de pouvoir se répliquer apparaît ainsi constituer l'un des principaux avantages des sondes de l'invention qui peuvent être obtenues de façon aisée et en très grand nombre.

L'invention entend protéger également tout fragment d'ADN différent de celui préféré indiqué plus haut par la substitution d'un ou deux nucléotides, cette substitution n'ayant pas pour effet de réduire la capacité d'hybridation de la séquence ainsi modifiée avec une séquence d'ADN complémentaire de la séquence non modifiée et de ne pas affecter la réplicabilité de l'ADN.

Constituent également des séquences analogues, celles s'hybridant avec le fragment préféré décrit plus haut, en formant un hybride stable et détectable dans les conditions opératoires qui seront décrites plus loin.

Il s'est avéré que la sonde selon l'invention peut contenir le motif unitaire tel que décrit ci-dessus répété un nombre $n$ fois, $n$ variant notamment d'environ 1 à environ 1 000, avantageusement d'environ 1 à environ 500.

La répétition des séquences s'effectue de façon monotone, la fin d'une séquence étant suivie du début de l'autre.

Il va de soi qu'une sonde formée par la séquence décrite plus haut, et répétée un nombre de fois plus grand encore, peut être mise en oeuvre pour réaliser les hybridations. Dans la pratique ces sondes seront moins avantageuses car elles ne donneraient que des images d'une qualité moyenne et difficilement exploitables.

Une sonde préférée selon l'invention comprend le fragment Hae III-Cla I issu du génome du bactériophage M13. Ce fragment de 280 paires de base contient un ADN mini-satellite, formé par la séquence préférée décrite plus haut, répétée de façon monotone neuf fois.

D'autres sondes préférées de l'invention sont constituées par le motif unitaire comprenant le fragment d'ADN tel que défini ci-dessus, répété un nombre $n$ de fois différent.

L'homme du métier sera à même de déterminer par de simples essais de routine le nombre de motifs unitaires nécessaires pour obtenir des résultats satisfaisants, notamment en fonction de la nature des différents échantillons à tester.

Selon une première variante d'utilisation, la sonde selon l'invention est utilisée telle quelle, le cas échéant la séquence nucléotidique constituant la sonde est incorporée dans un vecteur, notamment un plasmide ou un phage, distinct du phage M13 et ne possèdant pas de séquence similaire, ou présentant des homologies avec la séquence préférée décrite plus haut.

Il convient de noter à ce sujet que le bactériophage M13 entier constitue lui-aussi une sonde selon l'invention. En effet, le phage M13 contient dans son génome deux mini-satellites formés à partir de la séquence d'ADN décrite ci-dessus répétée en "tandem", ces mini-satellites étant présents dans le gène codant pour la protéine III du bactériophage M13.

Pour étonnant que ce résultat puisse paraître, l'on n'avait pas jusqu'à ce jour mis en évidence l'existence de séquences mini-satellites naturelles dans le génome du bactériophage M13. Ceci est peut être dû aux conditions dans lesquelles on réalise, habituellement, les expériences d'hybridation mettant en jeu le phage M13 classiquement utilisé à titre de vecteur de réplication et contenant, inséré dans l'une de ses régions non essentielles, la séquence-sonde intervenant dans l'hybridation avec une séquence complémentaire ou du même type dont, par exemple, la présence est recherchée dans une composition d'ADN à étudier préalablement fixée sur un support. Il est alors classique de réaliser ces opérations d'hybridation au sein de milieux qui sont saturés en ADNs étrangers à la réaction, notamment des ADNs de poisson, par exemple l'ADN de sperme de saumon ou de hareng. Cet excès d'ADN a en effet pour but de réduire les phénomènes de bruit de fond dûs, par exemple, à une hybridation d'une partie seulement de la sonde utilisée avec un fragment d'ADN complémentaire ou à des adsorptions ou fixations parasites de la sonde utilisée sur le support, notamment quand il s'agit de papier. L'excès d'ADN permet alors de réduire ces phénomènes, notamment en saturant les éventuels sites de fixation du support. Les ADNs en excès ne pouvaient dès lors que masquer l'intervention éventuelle de séquences particulières propres au phage M13 - à supposer même qu'on ait pu, antérieurement à l'invention, envisager leur existence - dans les essais d'hybridations mettant en jeu des sondes contenant des séquences nucléiques comprenant des mini-satellites, insérés dans ce phage M13 utilisé comme vecteur de réplication, en vue de détecter des liens de parentés éventuels entre les individus humains ou animaux dont provenaient les ADNs étudiés. En effet les ADNs, en excès, possèdent eux aussi des séquences mini-satellites susceptibles d'entrer en compétition avec les séquences particulières naturelles du bactériophages M13, séquences particulières dont le caractère de "séquences mini-satellites" a maintenant été mis en évidence dans le cadre de l'invention.

L'incorporation éventuelle des séquences-sondes selon l'invention dans un vecteur approprié, notamment un plasmide ou un phage distinct du phage M13, ne pose aucun problème que l'homme du métier avec ses connaissances ne saurait résoudre.

L'invention concerne également un procédé de détection de séquences d'ADN polymorphes utilisant les sondes de l'invention.

Le procédé selon l'invention pour la détection de séquences d'ADN polymorphes humaines, animales et/ou végétales, comprenant la mise en contact de la composition d'ADN à étudier dénaturée et fixée sur un support avec une sonde nucléotidique, dans des conditions permettant leur hybridation mutuelle éventuelle, se caractérise en ce que la sonde est constituée par un ADN issue du bactériophage M13, ladite sonde contenant n motifs unitaires répétés de façon monotone, chacun de ces motifs contenant la séquence nucléotidique suivant:

G-A-G-G-G-T-G-G-X-G-G-X-T-C-T

dans laquelle X représente la cytosine, l'adénine, la thymine ou la guanine, avantageusement la thymine ou la cytosine, en ce que le milieu d'hybridation est dépourvu d'ADN de compétition, et en ce que l'on détecte les "images" d'hybridation produites par les quantités d'ADN de phage hybridé et les localisations de la sonde sur l'ADN étudié.

De préférence le nombre n de motifs unitaires varie de 1 à 1 000, avantageusement de 1 à 500.

Avantageusement, le procédé de l'invention se caractérise en ce que la sonde est constituée par un ADN réplicable issu du bactériophage M13.

Le cas échéant, l'hybridation pourra être réalisée en présence d'un excès d'ADN étranger pour réduire le "bruit de fond" du genre sus-indiqué, cependant sous réserve de s'assurer de façon constante que cet ADN étranger soit lui-même dépourvu de séquences mini-satellites polymorphes, susceptibles d'entrer en compétition avec les régions mini-satellites de la sonde, et pouvant alors conduire à une inhibition de la sonde pour la détection de séquences nucléotidiques polymorphes, à tout le moins à des résultats difficilement exploitables.

Une forme de réalisation préférée du procédé de l'invention met en oeuvre un milieu capable de réduire de façon satisfaisante les bruits de fond propres à l'hybridation, tout en étant dépourvu de tout ADN en excès.

Avantageusement, le milieu d'hybridation est un milieu à base de lait en poudre écrémé. Un milieu préféré est le milieu Blotto, tel que décrit par Johnson D.A, (Gene. Anal. Tecn. (1984), 3-5).

Un second milieu d'hybridation préféré est un milieu à base d'héparine, tel que décrit par L.SINGH et al. Nucleic. Acid. Research. 12,(14), 5627-5638,(1984).

La détection des séquences hybridées est facilitée par le marquage de la sonde. Avantageusement, la sonde est marquée radioactivement avec un isotope radioactif, tel que l'isotope $^{32}$p du phosphore. Il va de soi que tout autre mode de marquage peut être utilisé (marquage enzymatique, fluorescent, etc...), qui, à condition d'être utilisé dans les conditions bien connues de l'homme du métier, doit aboutir à des sensibilités de détection semblables.

Selon un mode de réalisation préféré du procédé de l'invention, les supports sur lesquels sont fixés les ADN ou fragments d'ADN à tester, sont lavés plusieurs fois après l'hybridation avec une solution de chlorure de sodium et de citrate de sodium, avant d'être exposés à un moyen de révélation approprié en vue de la lecture des résultats.

Un premier moyen de révélation consiste à exposer les supports sur lesquels sont fixés les ADN ou fragments d'ADN à un film photographique dans le cas où la sonde est marquée radioactivement. Il va de soi que d'autres moyens de révélation sont utilisables, en fonction de la nature du marquage de la sonde. Il pourra s'agir par exemple d'une réaction enzymatique dans le cas où la sonde est marquée de façon enzymatique, par exemple avec la peroxidase.

La concentration de cette solution de lavage est couramment exprimée par l'abréviation SSC, dans laquelle une concentration de 1 SSC correspond à des concentrations en chlorure de sodium de 0,15 M et en citrate de sodium de 0,015 M dans une solution à un pH de 7,6.

D'autres solutions peuvent être utilisées avec la même efficacité pour le lavage des supports, à la condition toutefois qu'elles présentent le même degré de salinité que la solution préférée décrite ci-dessus.

Selon une forme de réalisation particulièrement préférée du procédé de l'invention, le dernier lavage avant exposition est réalisé avec une solution dont la concentration varie entre environ 0,1 SSC et environ 3 SSC.

Avantageusement, cette concentration est d'environ 1 SSC pour permettre d'abaisser le niveau du bruit de fond à un niveau tel que l'on obtienne des images d'une qualité suffisante pour leur exploitation.

Selon une forme de réalisation particulièrement préférée du procédé de l'invention, la température à laquelle est effectuée ce dernier lavage varie entre environ 40°C et 80°C, avantageusement est de 65°C.

Il est naturellement dans la nature des applications envisagées pour le procédé selon l'invention de mettre en oeuvre celui-ci dans des opérations de comparaison.

En d'autres termes, le procédé tel qu'il a été défini plus haut implique en général sa répétition sur au moins deux échantillons d'ADN provenant d'au moins deux individus (qui, le cas échéant, n'en formeront qu'un seul, s'agissant par exemple de comparer les identités d'une personne accusée d'un crime et de l'individu duquel provenait les traces de sang retrouvées sur les lieux du crime) et, le cas échéant aussi, d'un ADN témoin permettant de vérifier le caractère intact ou l'état de conservation de la sonde. Le procédé selon l'invention, réalisé dans ces conditions, implique alors encore la réalisation d'une étape de comparaison entre les "images" respectivement obtenues selon des critères eux-mêmes connus, par exemple ceux envisagés dans les articles référencés en (3) et (4).

L'invention concerne encore tous "kits" ou nécessaires comprenant respectivement :
- une sonde telle que définie ci-dessus,
- un milieu d'hybridation dépourvu de tout ADN de compétition tel que défini ci-dessus, et
- un fragment d'ADN témoin, servant à vérifier l'état de conservation de la sonde.

D'autres avantages et caractéristiques de l'invention apparaîtront encore dans les exemples qui suivent relatifs à l'isolement de la séquence nucléotidique de l'invention et à son utilisation en tant que sonde dans le procédé de l'invention, ainsi que dans les figures qui s'y rattachent dans lesquelles :
- La figure 1 représente les empreintes obtenues avec un échantillon d'ADN humain et l'ADN du bactériophage sauvage M13 utilisé en tant que sonde.
- La figure 2 représente l'identification de séquences appartenant au génome du bactériophage M13, s'hybridant avec un échantillon d'ADN de sperme de hareng.
- La figure 3a est une représentation schématique de la carte de restriction du bactériophage M13 montrant la localisation des sites de restriction Cla I, Bst N1, Xmn I, et Hae III représentés respectivement par les symboles ▼, ◆, ▽ et ◇.
- La figure 3b est une représentation schématique du mini-satellite isolé dans le gène de la protéine III du bactériophage M13.
- La figure 4 représente les empreintes détectées avec des échantillons d'ADN humain (a) et bovin (b) par hybridation avec le fragment d'ADN de 280 paires de base isolé dans le bactériophage M13.

### 1- Détection de polymorphismes dans un échantillonage d'ADN humain provenant de différents individus.

5 microgrammes d'ADN extraits de leucocytes humains sont digérés avec l'enzyme Hae III dans les conditions prescrites par les fabricants (Amersham-Boehringer). L'ADN ainsi fragmenté est soumis ensuite à une électrophorèse sur gel d'agarose à 1 % avec du tris-acétate 40 mM, de l'EDTA 1 mM, pH 7,7, à une tension constante d'environ 9 volts/centimètre pendant environ 4 heures. Les gels sont plongés dans une solution de NaCl 1,5 M, NaOH 0,5 M pendant 2 fois 30 minutes, sont mis ensuite en équilibre dans une solution d'acétate d'ammonium 1 M, NaOH, 0,03 M (2 x 30 minutes) et subissent un transfert pendant la nuit sur des filtres de nitrocellulose (Schleicher et Schell) dans la même solution.

200 ng du bactériophage M13 simple brin sont marqués avec du phosphore ($^{32}$p) radioactif, à une activité spécifique de 0,5 à 1,0 x $10^9$ coups.minutes$^{-1}$.µg$^{-1}$ selon la méthode décrite par Feinberg, A.P. et al, (Analyt. Biochem (1983) 132, 6-13).

Après une pré-hybridation de 2 heures, les filtres sont hybridés pendant la nuit à 42°C dans les solutions suivantes :
a et b : 40 % formamide, 6 SSC, EDTA 5 mM, 0,25 % de lait en poudre écrémé,
c : on utilise le même milieu mais le lait en poudre est remplacé par un milieu de Denhardt 5 x et 100 µg/ml d'ADN de sperme de hareng dénaturé.

Les filtres sont tous lavés dans les mêmes conditions :

- un premier lavage à 2 SSC, 0,1 % SDS (dodecylsulfate de sodium), à température ambiante pendant 2 x 15 minutes,
- un second lavage à 2 SSC, 0,1 SDS, à 65°C pendant 4 x 15 minutes,
- un dernier lavage à 1 SSC, à 65°C pendant 2 x 30 minutes.

Les filtres sont ensuite exposés à des films Kodak XAR-5 à -70°C avec un écran intensifiant pendant une durée allant de 16 heures à quelques jours.

On utilise pour cette expérience des échantillons d'ADN provenant de 11 individus de race caucasienne. Les individus 1 à 9 n'ont aucun lien de parenté entre eux. Les individus 10 et 11 sont des jumeaux monozygotes.

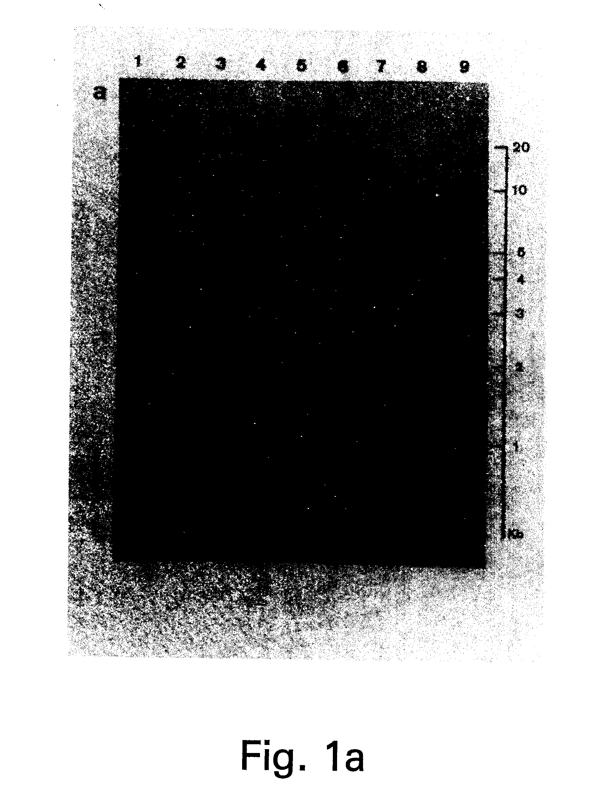

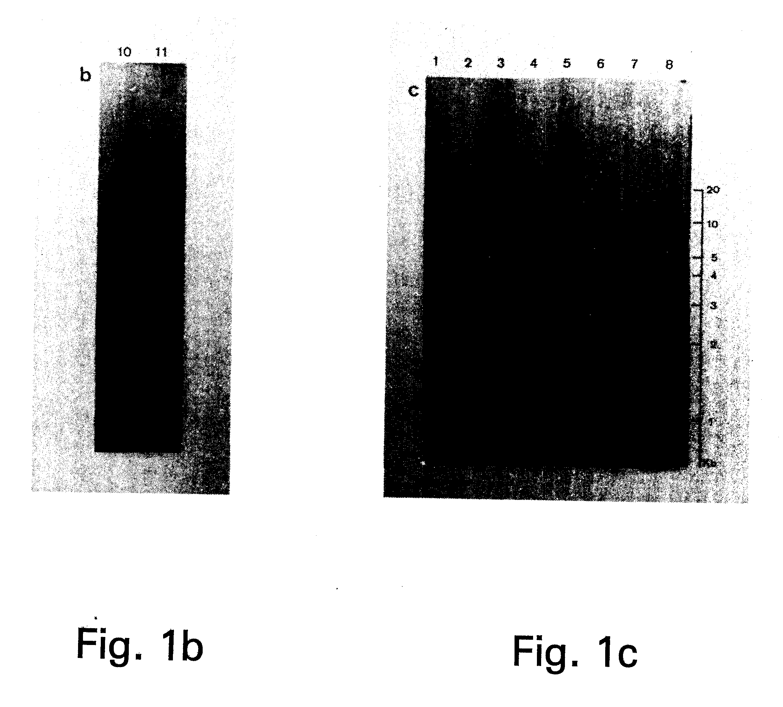

Les résultats expérimentaux sont montrés sur la figure 1. Il apparait sur celle-ci que, dans le milieu à base de lait en poudre correspondant aux cas a et b, les bandes d'hybridation obtenues pour les individus 1 à 9 sont toutes différentes, par contre celles obtenues avec les individus 10 et 11 sont identiques.

L'expérience identique réalisée avec un milieu différent (milieu de Denhardt et ADN de sperme de poisson) donne des profils de bandes identiques pour les individus 1 à 8, ne permettant aucune différenciation.

### 2- Isolement de la partie du génome du bactériophage M13 s'hybridant à l'ADN de sperme de hareng.

Les résultats expérimentaux obtenus dans l'expérience précédente ont permis d'isoler une région du génome du bactériophage M13, utilisable en tant que sonde pour la détection de séquences d'ADN polymorphes.

Cette partie du génome du bactériophage M13 a été isolée par la méthode décrite ci-après.

200 ng de l'ADN du phage M13 dans sa forme réplicative sont digérés avec différentes endonucléases (Xmn I, Bst N1 et Cla I).

Deux répliques par transfert identiques sont préparées par la méthode décrite précédemment, afin d'obtenir 2 filtres identiques.

Le filtre 'a' est hybridé avec une sonde constituée du phage M13 entier marqué radioactivement par la méthode référencée précédemment.

Le filtre 'b' est hybridé avec des séquences issues du génome du bactériophage M13 marquées radioactivement, qui ont été sélectionnées par hybridation à de l'ADN de sperme de hareng tel que décrit ci-après.

Les deux filtres sont hybridés et lavés dans les mêmes conditions que précédemment.

La sélection des séquences issues du génome du bactériophage M13 s'hybridant avec l'ADN de sperme de hareng est réalisée de la manière suivante : un filtre de nitrocellulose de 100 cm² est saturé avec de l'ADN de sperme de hareng en le trempant pendant 30 minutes dans une solution d'acétate d'ammonium 1 M, NaOH 0,03 M contenant 2,5 mg/ml l'ADN de sperme de hareng dénaturé et renforcé à 80°C pendant 2 heures.

Le filtre est pré-hybridé pendant 2 heures dans 40 % formamide, 6 SSC, EDTA 5 mM, 0,25 % de lait en poudre à 42°C, et mis à hybrider pendant la nuit avec une sonde consistant en de l'ADN marqué au phosphore radioactif [32]p issu du bactériophage M13 entier.

Le filtre est ensuite lavé tel qu'il a été décrit ci-dessus.

Les séquences issues du génome du bactériophage M13 sont ensuite éluées après avoir fait bouillir le filtre pendant 10 minutes dans 10 ml de 10 mM tris-HCl, pH 8, 1 mM EDTA. Environ 0,1 % de la radioactivité du départ est retrouvé.

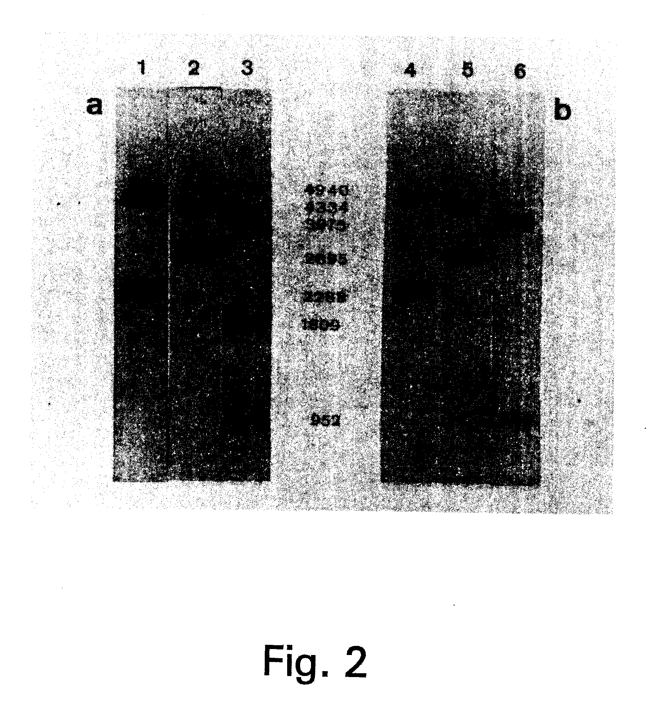

Les résultats expérimentaux sont illustrés sur la figure 2.

Le fragment d'ADN du bactériophage M13 dans sa forme réplicative est digéré avec les enzymes Xmn I (lignes 1,4), Bst N1 (lignes 2,5) et Cla I (lignes 3,6).

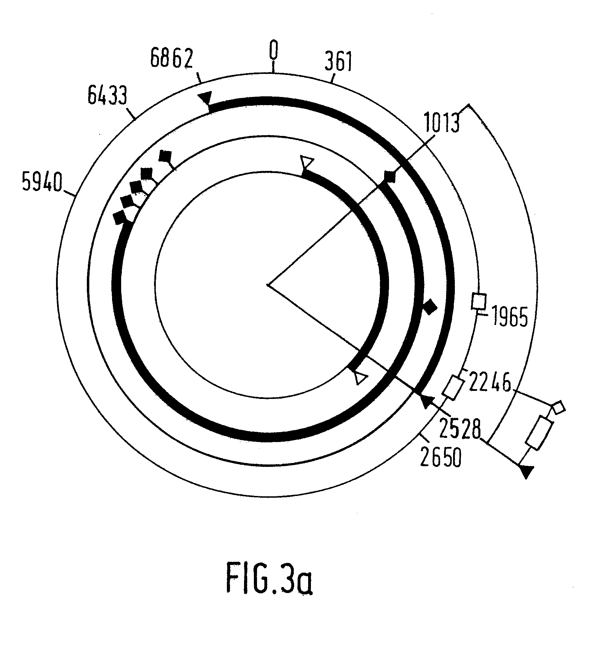

Les résultats expérimentaux indiquent que la sonde purifiée est enrichie en séquences correpondant à une région se situant entre les positions 1013 et 2528 telles qu'indiquées sur la carte de restriction du bactériophage M13 (figure 3a).

Sur cette figure 3a sont indiqués les sites de reconnaissance des enzymes Cla I, Bst N1, Xmn I et Hae III, respectivement représentés par les symboles ▼, ◆, ▽, et ◇.

Les lignes en trait gras montrent les fragments de restriction qui sont reconnus préférentiellement par les séquences purifées du bactériophage M13. Les deux rectangles donnent la localisation des deux ADN minisatellites répétées en tandem.

L'étude de la région du bactériophage M13 située entre les coordinats 1013 et 2528 a révélé la présence d'un motif de 15 paires de base répété en tandem à deux endroits dans le gène de la protéine III du pahge M13.

Ce motif de 15 paires de base correspond à la séquence : Glu-Gly-Gly-Gly-Ser.

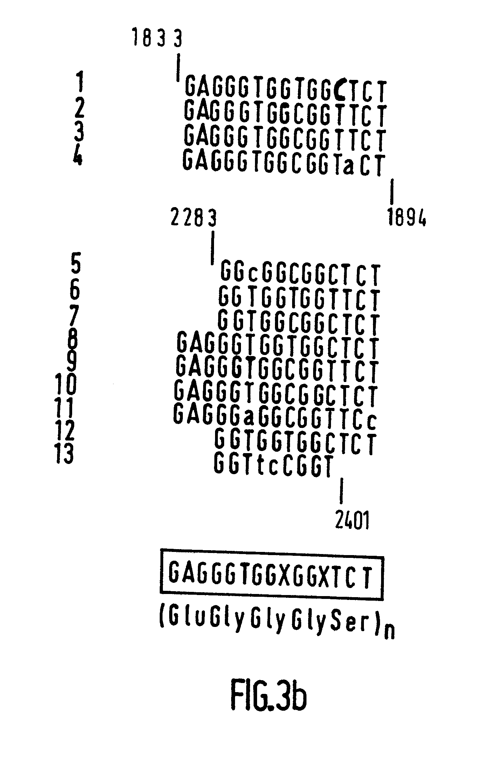

On a représenté sur la figure 3b l'alignement des séquences répétées en tandem isolées dans le gène de la protéine III du bactériophage M13. Les coordinats sont de Van Wezembeck, P.M. et al , (Gene. 11 (1980) 129-148).

3- Exemples de détection de séquences polymorphes issues de fragments d'ADN humain et bovin.

Le fragment de restriction Hae III-Cla I de 280 paires de base du bactériophage M13, tel que représenté sur la figure 3a, est obtenu après digestion par les enzymes de restriction appropriées, électrophorèse sur gel d'agarose et électroélution. 100 ng de ce fragment sont marqués avec du phosphore [32]p radioactif, à une activité spécifique de 0,5 - 1 x 10$^9$ coups./minutes$^{-1}$. µg$^{-1}$ selon la méthode référencée précédemment.

Cette sonde est utilisée dans les expériences mettant en jeu la technique dite de "Southern Blotting" telle que décrite plus haut en utilisant le milieu à base de lait en poudre pour l'hybridation.

Il va de soi que l'on aurait pu utiliser de la même façon le fragment génomique du bactériophage M13 situé entre les coordinats 1833 et 1894, tel qu'indiqué sur la figure 3b.

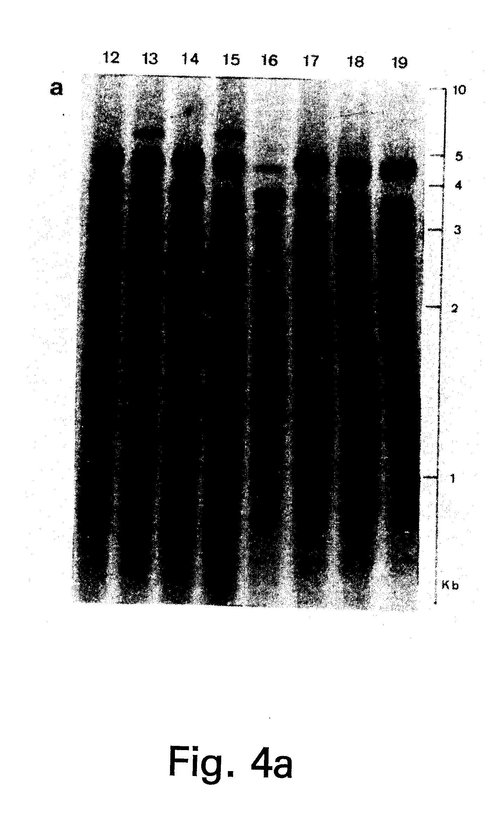

Sur la figure 4a, on a représenté les résultats obtenus avec des échantillons d'ADN humain. Les individus numérotés de 12 à 17 sont de race caucasienne et issus de la même famille, les individus 18 et 19 sont des jumeaux monozygotes.

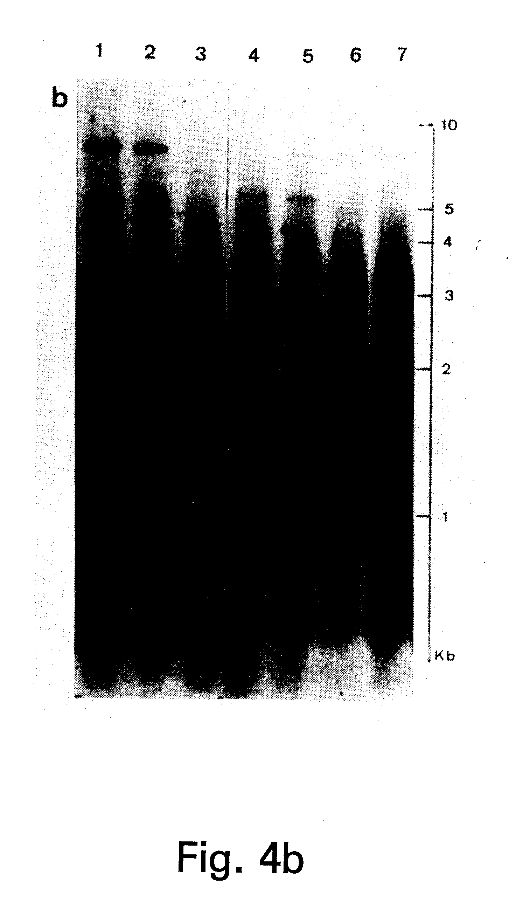

La figure 4b représente les résultats obtenus avec des échantillons d'ADN bovin. Les individus 1 et 2 sont des embryons de jumeaux monozygotes obtenus à partir d'une morula de bovin. Les individus 3 à 7 sont des animaux sans lien de parenté, de race "blanc-bleue-belge".

L'invention telle que décrite précédemment s'applique particulièrement bien à des analyses de pedigree dans les espèces humaines et animales, par exemple bovidé, cheval, ovidé, chien, etc... Ces analyses de pedigree sont également applicables pour les recherches de paternité ou d'identification d'un individu à partir d'un aliquot d'ADN.

L'invention s'applique également à l'étude des relations éventuelles existant entre certaines régions génomiques polymorphes et certaines maladies, aussi bien pour l'homme que pour l'animal.

L'invention s'applique également aux études des relations éventuelles existant entre les régions polymorphes et le caractère hériditaire, notamment pour l'élevage.

Il va de soi que l'invention ne se limite nullement aux exemples de réalisation qui ont été décrits plus particulièrement ci-dessus. Elle en embrasse au contraire toutes les variantes.

REFERENCES

1. JEFFREYS A.J et al, Nature (1985), 314, p.67-73

2. GOODBOURN S.E.Y et al, Proc. Natl. Acad. Sci. USA (1983), 80, 5022-5026

3. GILL P. et al Nature (1985), 318, 577-579

4. JEFFREYS A.J. et al, Nature (1985), 317, 818-819

5. SANGER F. et al, J. Mol. Biol. (1980), 143, 161-178

**Revendications**

1. Sonde nucléotidique pour la détection de séquences d'ADN polymorphes humaines, animales et/ou végétales, caractérisée en ce qu'elle est constituée par un ADN issu du bactériophage M13 et en ce qu'elle contient une séquence formée d'au moins un motif unitaire répété de façon monotone, chaque motif étant constitué par une séquence nucléotidique contenant la séquence suivante : G-A-G-G-G-T-G-G-X-G-G-X-T-C-T dans laquelle X représente la thymine, la cytosine, l'adénine ou la guanine, avantageusement la thymine ou la cytosine.

2. Sonde nucléotidique selon la revendication 1, caractérisée en ce qu'elle est constituée par un ADN réplicable issu du bactériophage M13.

3. Sonde selon la revendication 1 ou 2, caractérisée en ce qu'elle comprend le fragment Hae III-Cla I du génome du bactériophage M13.

4. Sonde selon l'une quelconque des revendications 1 ou 3, caractérisée en ce qu'elle est formée par un vecteur distinct du bactériophage M13, notamment un plasmide ou un phage, dans lequel la susdite séquence est incorporée, ce vecteur étant lui-même, de préférence, dépourvu des séquences endogènes présentant des homologies avec les susdites séquences.

5. Procédé de détection de séquences d'ADN polymorphes humaines, animales et/ou végétales comprenant la mise en contact de la composition d'ADN à étudier dénaturée et fixée sur un support avec une sonde nucléotidique, dans des conditions permettant leur hybridation mutelle éventuelle, caractérisé en ce que la sonde est constituée par un ADN issu du bactériophage M13, ladite sonde contenant $n$ motifs unitaires répétés de façon monotone, $n$ variant de préférence de 1 à 1 000, avantageusement de 1 à 500, chacun de ces motifs contenant la séquence nucléotidique suivante :

G-A-G-G-G-T-G-G-X-G-G-X-T-C-T

dans laquelle $X$ représente la cytosine, l'adénine, la thymine ou la guanine, avantageusement la thymine ou la cytosine, en ce que le milieu d'hybridation est dépourvu d'ADN de compétition, et en ce que l'on détecte les "images" d'hybridation produites par les quantités d'ADN de phage hybridé et les localisations de la sonde sur l'ADN étudié.

6. Procédé selon la revendication 5, caractérisé en ce que la sonde est constituée par un ADN réplicable issu du bactériophage M13.

7. Procédé selon la revendication 5 ou 6, caractérisé en ce que la sonde est marquée.

8. Procédé selon l'une quelconque des revendications 5 à 7, appliqué à des échantillons d'ADN distincts, caractérisé en ce que l'on compare les images d'hybridation obtenues avec chacun des ces échantillons et en ce que l'on détermine le degré de parenté, voire même d'identité, des individus desquels provenaient ces échantillons d'ADN.

9. Procédé selon l'une quelconque des revendications 5 à 8, caractérisé en ce que le milieu d'hybridation est un milieu à base de lait ou à base d'héparine.

10. Procédé selon l'une quelconque des revendications 5 à 9, caractérisé en ce que l'on effectue un lavage de la composition d'ADN à étudier après hybridation à une concentration variant d'environ 0,1 SSC à environ 3 SSC, avantageusement à 1 SSC.

11. Procédé selon la revendication 10, caractérisé en ce que le lavage est effectué à une température variant d'environ 40°C à environ 80°C, avantageusement à 65°C.

12. Kit pour la détection de séquences d'ADN polymorphes humaines, animales et/ou végétales, caractérisé en ce qu'il comprend :
- une sonde selon l'une quelconque des revendications 1 à 4,
- un milieu d'hybridation dépourvu d'ADN de compétition,
- un échantillon d'ADN témoin.

0264305

Fig. 1a

0264305

Fig. 1b

Fig. 1c

0264305

Fig. 2

0264305

# FIG.3a

0264305

1833

1 GAGGGTGGTGGCTCT
2 GAGGGTGGCGGTTCT
3 GAGGGTGGCGGTTCT
4 GAGGGTGGCGGTaCT

1894

2283

5 GGcGGCGGCTCT
6 GGTGGTGGTTCT
7 GGTGGCGGCTCT
8 GAGGGTGGTGGCTCT
9 GAGGGTGGCGGTTCT
10 GAGGGTGGCGGCTCT
11 GAGGGaGGCGGTTCc
12 GGTGGTGGCTCT
13 GGTtcCGGT

2401

GAGGGTGGXGGXTCT

(GluGlyGlyGlySer)n

FIG.3b

Fig. 4a

0264305

Fig. 4b